# EUROPEAN PATENT APPLICATION

(11) **EP 4 406 545 A1**
(43) Date of publication of application: **31.07.2024**
(21) Application number: 23154012.1
(22) Date of filing: 30.01.2023
(51) Int. Cl.: A61K 35/26, A61K 35/17, A61P 27/02

(54) **A METHOD OF PRODUCING INNATE LYMPHOID CELLS FOR USE IN THERAPY**

(71) Applicant: The Provost, Fellows, Scholars and other Members of Board of Trinity College Dublin, Dublin 2 (IE)
(72) Inventor: Doyle, Sarah, Dublin, 2 (IE); Campbell, Matthew, Dublin, 2 (IE); Ozaki, Ema, Dublin, 2 (IE)
(74) Representative: FRKelly

(57) **Abstract**

The present invention relates to a method of producing a population of activated innate lymphoid cells, the population of activated innate lymphoid cells obtainable thereby, and use thereof in preventing or treating a disease in a subject. The present invention finds particular utility in the production of activated innate lymphoid cells useful in the treatment of ocular diseases such as neovascular diseases of the choroid and/or the retina.

## Description

### Field of the invention

The present invention relates to a method of producing a population of activated innate lymphoid cells, the population of activated innate lymphoid cells obtainable thereby, and use thereof in preventing or treating a disease in a subject.

### Background to the invention

The retina is an extension of the central nervous system, nourished by two vascular beds, the choroid and the inner retinal vasculature. In order to maintain its function in transmitting accurate visual information, the retina requires a highly organised laminar structure. Consequently, like the brain, the microenvironment of the retina is tightly regulated and, as an immune privileged tissue, is practically devoid of infiltrating immune cells in the sub-retinal space of its outer layers. However, immune cells can and do infiltrate the sub-retinal space in retinal disease and there are many reports examining retinal degeneration coinciding with the appearance of innate myeloid cells in retinal tissue.

Despite different underlying triggers of disease, uncontrolled vascular permeability and pathological vessel neovascularization are common pathologies for a variety of retinal diseases, including Age-related Macular Degeneration (AMD), Retinopathy of Prematurity (ROP), Diabetic Retinopathy (DR), Diabetic Macular Oedema (DME), and Branch Retinal Vein Occlusion.

Globally, AMD and DR combined are the most common diseases of the retina that lead to sight loss.

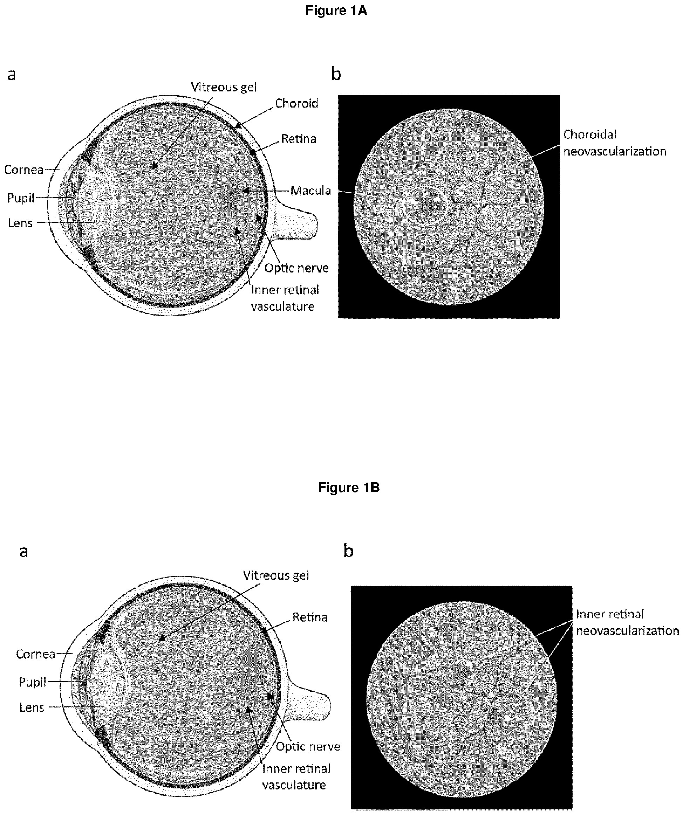

AMD is a chronic disease and, distressingly for the ~200 million people affected, treatment is limited by an incomplete understanding of the mechanisms that trigger pathology. The macula is situated centrally at the back of the eye, and while occupying only 4% of the surface area of the retina, it is responsible for 90% of vision. Therefore, it is the central vision that is lost in AMD. AMD can present in two forms, dry AMD and neovascular or 'wet' AMD, and there are two end stages of the disease. The end stage of dry AMD is termed Geographic Atrophy (GA) and is characterized by atrophy of the Retinal Pigment Epithelium (RPE) cell layer that supports the retina. The end stage of wet AMD is characterized by the growth of abnormal new blood vessels originating from the choroid, termed choroidal neovascularization (CNV). These new vessels penetrate Bruch's membrane at the macula and grow within the sub-RPE or sub-retinal space leaking exudative material, which results in vision loss. Approximately 20% of individuals diagnosed with AMD will develop late stage disease. Of those who progress to late stage, 70% will develop neovascular AMD (nvAMD) termed a priority eye disease by the World Health Organization (see Figure 1A).

Whereas AMD is the principal cause of central vision loss in individuals over 50 worldwide, diabetic retinopathy (DR) is the leading cause of legal blindness in working-age adults in the western world. The International Diabetes Federation (IDF) released figures in December 2021 showing that more than half a billion adults are now living with diabetes worldwide, an alarming rise of 16% (74 million) since the previous IDF estimates. Inevitably, 90% of people with type-1 diabetes and nearly 80% with type 2 diabetes for over 10 years will develop DR caused by the systemic elevated blood glucose that can lead to damage of the retinal microvasculature. Hallmarks of DR include formation of capillary micro-aneurysms, development of ischemic zones within the retina, inner retinal capillary leakage and subsequent exudation and oedema. The tissue ischemia ultimately promotes retinal neovascularization characterised by the growth of new retinal and pre-retinal fronds (see Figure 1B).

Treatment for nvAMD and DR involves regular injection of "anti-VEGF" biologics directly into the eye. While anti-VEGF therapy has revolutionized the outcome for patients with nvAMD, it is not without its limitations, and it has been substantively less successful for treating DR. Anti-VEGF therapy efficiently prevents the hyper-permeability of developing CNV lesions. However, it does not promote regression of these lesions and can ultimately lead to extensive fibrotic scarring. Emerging long term studies indicate that -50% of patients treated with anti-VEGF develop resistance to treatment within 2 years of first treatment, due to onset of sub-retinal fibrosis (end-stage disease). Added to this, a significant proportion of nvAMD and DR patients are refractory to anti-VEGF treatment from disease onset, estimated at approximately 25-40% for nvAMD and 15-20% for DR. With human populations now living longer than at any other time in history, and the accelerating rise in both AMD and incident diabetes, there is a very real need for new therapies for the management of retinal neovascular disease, which represents a major challenge of our time.

### Summary of the invention

According to a first aspect of the present invention, there is provided a method of producing a population of activated innate lymphoid cells, the method comprising the steps of:
(a) providing a population of innate lymphoid cells; and
(b) contacting the population of innate lymphoid cells with at least one cytokine.

According to a second aspect of the present invention, there is provided use of at least one cytokine in the production of a population of activated innate lymphoid cells, the use comprising the steps of:
(a) providing a population of innate lymphoid cells; and
(b) contacting the population of innate lymphoid cells with the cytokine.

According to a third aspect of the present invention, there is provided a population of activated innate lymphoid cells, optionally obtainable by providing a population of innate lymphoid cells and contacting the population of innate lymphoid cells with at least one cytokine.

According to a fourth aspect of the present invention, there is provided a method of activating a population of innate lymphoid cells, the method comprising the steps of:
(a) providing a population of innate lymphoid cells; and
(b) contacting the population of innate lymphoid cells with at least one cytokine.

According to a fifth aspect of the present invention, there is provided use of at least one cytokine in the activation of a population of innate lymphoid cells, the use comprising:
(a) providing the population of innate lymphoid cells; and
(b) contacting the population of innate lymphoid cells with the cytokine.

According to a sixth aspect of the present invention, there is provided a method of preventing or treating a disease in a subject, the method comprising the steps of:
(a) providing a population of innate lymphoid cells;
(b) contacting the population of innate lymphoid cells with at least one cytokine to produce a population of activated innate lymphoid cells; and
(c) administering the population of activated innate lymphoid cells or a pharmaceutical composition comprising the population of activated innate lymphoid cells, to the subject.

According to a seventh aspect of the present invention, there is provided a population of activated innate lymphoid cells, optionally obtainable by providing a population of innate lymphoid cells and contacting the population of innate lymphoid cells with at least one cytokine, for use in therapy.

According to a eighth aspect of the present invention, there is provided a population of activated innate lymphoid cells for use in therapy, the use comprising:
(a) providing a population of innate lymphoid cells;
(b) contacting the population of innate lymphoid cells with at least one cytokine to produce a population of activated innate lymphoid cells; and
(c) administering the population of activated innate lymphoid cells or a pharmaceutical composition comprising the population of activated innate lymphoid cells, to the subject.

According to a ninth aspect of the present invention, there is provided use of a population of activated innate lymphoid cells, optionally obtainable by providing a population of innate lymphoid cells and contacting the population of innate lymphoid cells with at least one cytokine, in the manufacture of a medicament for preventing or treating a disease in a subject.

According to a tenth aspect of the present invention, there is provided use of a population of activated innate lymphoid cells in the manufacture of a medicament for preventing or treating a disease in a subject, the use comprising:
(a) providing a population of innate lymphoid cells;
(b) contacting the population of innate lymphoid cells with at least one cytokine to produce a population of activated innate lymphoid cells; and
(c) administering the population of activated innate lymphoid cells or a pharmaceutical composition comprising the population of activated innate lymphoid cells, to the subject.

According to an eleventh aspect of the present invention, there is provided a population of activated innate lymphoid cells.

According to an twelfth aspect of the present invention, there is provided a pharmaceutical composition comprising a population of activated innate lymphoid cells and a pharmaceutically acceptable carrier.

Optionally, the at least one cytokine is at least one proinflammatory cytokine.

Optionally, the at least one cytokine is selected from interleukin 18 (IL-18), interleukin 12 (IL-12), interleukin 15 (IL-15), interleukin 2 (IL-2), and combinations each thereof.

Optionally, the at least one cytokine is a combination of IL-18, IL-12, and IL-15.

Optionally, the at least one cytokine is a combination of IL-18 and IL-12.

Optionally, the at least one cytokine is a combination of IL-12 and IL-2.

Optionally, the at least one cytokine is a combination of IL-15 and IL-2.

Optionally, the at least one cytokine is IL-18.

Preferably, the at least one cytokine is IL-18.

Optionally, the step of contacting the population of innate lymphoid cells with at least one cytokine is conducted for at least 3 hours, optionally at least 6 hours, optionally at least 9 hours, optionally at least 12 hours, optionally at least 15 hours, optionally at least 18 hours, optionally at least 21 hours, optionally at least 24 hours, optionally at least 27 hours, optionally at least 30 hours, optionally at least 33 hours, optionally at least 36 hours.

Preferably, the step of contacting the population of innate lymphoid cells with at least one cytokine is conducted for at least 18 hours.

Optionally, the method or use comprises the additional step of expanding the population of innate lymphoid cells.

Optionally, the method or use comprises the additional step of contacting the population of innate lymphoid cells with at least one cytokine to expand the population of innate lymphoid cells.

Optionally, the at least one cytokine to expand the population of innate lymphoid cells is selected from IL-15 and IL-2.

Optionally, the at least one cytokine to expand the population of innate lymphoid cells is IL-2.

Preferably, the at least one cytokine to expand the population of innate lymphoid cells is IL-2.

Optionally, the step of expanding the population of innate lymphoid cells is conducted for at least 12 hours, optionally at least 1 day, optionally at least 2 days, optionally at least 3 days, optionally at least 4 days, optionally at least 5 days, optionally at least 6 days, optionally at least 7 days, optionally at least 8 days, optionally at least 9 days, optionally at least 10 days, optionally at least 11 days, optionally at least 12 days, optionally at least 13 days, optionally at least 14 days.

Preferably, the step of expanding the population of innate lymphoid cells is conducted for at least 7 days.

Optionally, the at least one cytokine is a human cytokine.

Optionally, the at least one cytokine is a naturally occurring cytokine.

Optionally, the at least one cytokine is a recombinant cytokine.

Optionally, the at least one cytokine is a naturally occurring human cytokine.

Optionally, the at least one cytokine is a recombinant human cytokine.

Optionally, the disease is an ocular disease.

Optionally, the disease is a vascular ocular disease.

Optionally, the disease is a neovascular ocular disease.

Optionally, the disease is a neovascular disease of the choroid and/or the retina.

Optionally, the disease is selected from age-related macular degeneration, retinopathy of prematurity, diabetic retinopathy, diabetic macular edema, and branch retinal vein occlusion.

Optionally, the disease is age-related macular degeneration.

Preferably, the disease is age-related macular degeneration.

Optionally, the disease is selected from dry (non-exudative) age-related macular degeneration and wet (neovascular) age-related macular degeneration.

Optionally, the disease is dry (non-exudative) age-related macular degeneration, optionally geographic atrophy.

Optionally, the disease is wet (neovascular) age-related macular degeneration, optionally choroidal neovascularization (CNV).

Optionally, the disease is diabetic retinopathy.

Optionally, the innate lymphoid cell is a lymphocyte.

Optionally, the innate lymphoid cell is a natural killer cell.

Preferably, the innate lymphoid cell is a natural killer cell.

Optionally, the method or use comprises the steps of:
(a) providing a population of natural killer cells; and
(b) contacting the population of natural killer cells with at least one cytokine to produce a population of activated natural killer cells.

Optionally, the method or use comprises the steps of:
(a) providing a population of natural killer cells; and
(b) contacting the population of natural killer cells with at least one cytokine selected from IL-18, IL-12, IL-15, IL-2, and combinations each thereof to produce a population of activated natural killer cells.

Preferably, the method or use comprises the steps of:
(a) providing a population of natural killer cells; and
(b) contacting the population of natural killer cells with IL-18 to produce a population of activated natural killer cells.

Optionally, the method or use comprises the steps of:
(a) providing a population of natural killer cells;
(b) contacting the population of natural killer cells with at least one cytokine to produce a population of activated natural killer cells; and
(c) administering the population of activated natural killer cells to the subject.

Optionally, the method or use comprises the steps of:
(a) providing a population of natural killer cells;
(b) contacting the population of natural killer cells with at least one cytokine selected from IL-18, IL-12, IL-15, IL-2, and combinations each thereof to produce a population of activated natural killer cells; and
(c) administering the population of activated natural killer cells to the subject.

Preferably, the method or use comprises the steps of:
(a) providing a population of natural killer cells;
(b) contacting the population of natural killer cells with IL-18 to produce a population of activated natural killer cells; and
(c) administering the population of activated natural killer cells to the subject.

Optionally, the cell is a mouse cell.

Optionally, the cell is a human cell.

Preferably, the cell is a human cell.

Optionally, the activated cell is in a phase of the cell cycle selected from interphase and the mitotic phase (mitosis).

Optionally, the activated cell is in a phase of the cell cycle selected from Gap 1 (G1), Synthesis (S), Gap 2 (G2) and the mitotic phase (mitosis, M).

Optionally, the activated cell is in a phase of the cell cycle selected from Gap 2 (G2) and the mitotic phase (mitosis, M).

Optionally, the activated cell is in the Gap 2/mitosis (G2/M) transition phase of the cell cycle.

Optionally, the activated cell has an expression of at least one gene, or gene expression product expressed thereby, selected from TOP2A; PCLAF; SMC2; ANXA1; B4GALT1; FCGRIII; ENTPD1; PTPRC; NCAM1; B3GAT1; CD69; NT5E; CYP26A1; CD226; FASLG; GNLY; GZMA; GZMB; ITGB2; MKI67; KIR2DL1; KIR3DL1; KLRB1; KLRK1; NCR3; NCR1; TNFSF10; and PRF1.

Optionally, the activated cell has an expression of at least one gene having an Ensembl transcript ID, or gene expression product expressed thereby, selected from ENSG00000131747; ENSG00000166803; ENSG00000136824; ENSG00000135046; ENSG00000086062; ENSG00000203747; ENSG00000138185; ENSG00000081237; ENSG00000149294; ENSG00000109956; ENSG00000110848; ENSG00000135318; ENSG00000095596; ENSG00000150637; ENSG00000117560; ENSG00000115523; ENSG00000145649; ENSG00000100453; ENSG00000160255; ENSG00000148773; ENSG00000125498; ENSG00000167633; ENSG00000111796; ENSG00000213809; ENSG00000204475; ENSG00000189430; ENSG00000121858; and ENSG00000180644.

Optionally, the activated cell has an expression of at least one gene having a Genbank accession/version number, or gene expression product expressed thereby, selected from NM_001067.4; NM_001029989.3; NM_014736.6; NM_001042551.2; NM_001265602.2; NM_006444.3; NM_001042550.2; NM_000700.3; NM_001378495.1; NM_001378496.1; NM_001497.4; NM_001378497.1; NM_000569.8; NM_001127595.2; NM_001127593.1; NM_001127592.2; NM_001329122.1; NM_001329120.2; NM_001127596.2; NM_001386450.1; NM_001098175.2; NM_001164178.1; NM_001320916.1; NM_001164181.1; NM_001164182.2; NM_001164183.2; NM_001312654.1; NM_001164179.2; NM_001776.6; NM_002838.5; NM 080921.4; NM_001267798.2; NM_001242608.2; NM_001400603.1; NM_001400606.1; NM_000615.7; NM_001386292.1; NM_001076682.4; NM_001386290.1; NM_001400615.1; NM_181351.5; NM_001242607.2; NM_001386289.1; NM_001386291.1; NM_001400618.1; NM_001400605.1; NM_001400616.1; NM_018644.3; NM_001367973.1; NM_054025.3; NM_001781.2; NM_001204813.2; NM_002526.4; NM_057157.2; NM_000783.4; NM_001303618.2; NM_006566.4; NM_001303619.2; NM_000639.3; NM_000639.3; NM_006433.5; NM_012483.4; NM_001302758.2; NM_006144.4; NM_004131.6; NM_001346011.2; NM_000211.5; NM_001303238.2; NM_001127491.3; NM_002417.5; NM_001145966.2; NM_014218.3; NM_013289.3; NM_002258.3; NM_001199805.1; NM_001199805.1; NM_001145466.2; NM_147130.3; NM_001145467.2; NM_001242356.3; NM_001242357.3; NM_001145458.3; NM_004829.7; NM_001145457.3; NM_003810.4; NM_001190942.2; NM_001190943.2; NM_001083116.3; and NM_005041.6.

Optionally, the activated cell has a higher expression of at least one gene, or gene expression product expressed thereby, relative to a non-activated cell (a cell not contacted with the at least one cytokine).

Optionally, the at least one gene, or gene expression product expressed thereby, is selected from TOP2A; PCLAF; SMC2; ANXA1; B4GALT1; FCGRIII; ENTPD1; PTPRC; NCAM1; B3GAT1; CD69; NT5E; CYP26A1; CD226; FASLG; GNLY; GZMA; GZMB; ITGB2; MKI67; KIR2DL1; KIR3DL1; KLRB1; KLRK1; NCR3; NCR1; TNFSF10; and PRF1.

Optionally, the at least one gene has an Ensembl transcript ID, or gene expression product expressed thereby, selected from ENSG00000131747; ENSG00000166803; ENSG00000136824; ENSG00000135046; ENSG00000086062; ENSG00000203747; ENSG00000138185; ENSG00000081237; ENSG00000149294; ENSG00000109956; ENSG00000110848; ENSG00000135318; ENSG00000095596; ENSG00000150637; ENSG00000117560; ENSG00000115523; ENSG00000145649; ENSG00000100453; ENSG00000160255; ENSG00000148773; ENSG00000125498; ENSG00000167633; ENSG00000111796; ENSG00000213809; ENSG00000204475; ENSG00000189430; ENSG00000121858; and ENSG00000180644.

Optionally, the at least one gene has a Genbank accession/version number, or gene expression product expressed thereby, selected from NM_001067.4; NM_001029989.3; NM_014736.6; NM_001042551.2; NM_001265602.2; NM_006444.3; NM_001042550.2; NM_000700.3; NM_001378495.1; NM_001378496.1; NM_001497.4; NM_001378497.1; NM_000569.8; NM_001127595.2; NM_001127593.1; NM_001127592.2; NM_001329122.1; NM_001329120.2; NM_001127596.2; NM_001386450.1; NM_001098175.2; NM_001164178.1; NM_001320916.1; NM_001164181.1; NM_001164182.2; NM_001164183.2; NM_001312654.1; NM_001164179.2; NM 001776.6; NM_002838.5; NM_080921.4; NM_001267798.2; NM_001242608.2; NM_001400603.1; NM_001400606.1; NM_000615.7; NM_001386292.1; NM_001076682.4; NM_001386290.1; NM_001400615.1; NM_181351.5; NM_001242607.2; NM_001386289.1; NM_001386291.1; NM_001400618.1; NM_001400605.1; NM_001400616.1; NM_018644.3; NM_001367973.1; NM_054025.3; NM_001781.2; NM_001204813.2; NM_002526.4; NM_057157.2; NM_000783.4; NM_001303618.2; NM_006566.4; NM_001303619.2; NM_000639.3; NM_000639.3; NM_006433.5; NM_012483.4; NM_001302758.2; NM_006144.4; NM_004131.6; NM_001346011.2; NM_000211.5; NM_001303238.2; NM_001127491.3; NM_002417.5; NM_001145966.2; NM_014218.3; NM_013289.3; NM_002258.3; NM_001199805.1; NM_001199805.1; NM_001145466.2; NM_147130.3; NM_001145467.2; NM_001242356.3; NM_001242357.3; NM_001145458.3; NM_004829.7; NM_001145457.3; NM_003810.4; NM_001190942.2; NM_001190943.2; NM_001083116.3; and NM_005041.6.

Optionally, the cell is a mouse cell and the at least one gene, or gene expression product expressed thereby, is a selected from TOP2A, PCLAF, and SMC2.

Optionally, the cell is a mouse cell and the at least one gene has an Ensembl transcript ID, or gene expression product expressed thereby, selected from ENSG00000131747; ENSG00000166803; and ENSG00000136824.

Optionally, the cell is a mouse cell and the at least one gene has a Genbank accession/version number, or gene expression product expressed thereby, selected from NM_001067.4; NM_001029989.3; NM_014736.6; NM_001042551.2; NM_001265602.2; NM_006444.3; and NM_001042550.2.

Optionally, the cell is a human cell and the at least one gene, or gene expression product expressed thereby, is a selected from ANXA1; B4GALT1; FCGRIII; ENTPD1; PTPRC; NCAM1; B3GAT1; CD69; NT5E; CYP26A1; CD226; FASLG; GNLY; GZMA; GZMB; ITGB2; MKI67; KIR2DL1; KIR3DL1; KLRB1; KLRK1; NCR3; NCR1; TNFSF10; and PRF1.

Optionally, the cell is a human cell and the at least one gene has an Ensembl transcript ID, or gene expression product expressed thereby, selected from ENSG00000135046; ENSG00000086062; ENSG00000203747; ENSG00000138185; ENSG00000081237; ENSG00000149294; ENSG00000109956; ENSG00000110848; ENSG00000135318; ENSG00000095596; ENSG00000150637; ENSG00000117560; ENSG00000115523; ENSG00000145649; ENSG00000100453; ENSG00000160255; ENSG00000148773; ENSG00000125498; ENSG00000167633; ENSG00000111796; ENSG00000213809; ENSG00000204475; ENSG00000189430; ENSG00000121858; and ENSG00000180644.

Optionally, the cell is a human cell and the at least one gene has a Genbank accession/version number, or gene expression product expressed thereby, selected from NM_000700.3; NM_001378495.1; NM_001378496.1; NM_001497.4; NM_001378497.1; NM_000569.8; NM_001127595.2; NM_001127593.1; NM_001127592.2; NM_001329122.1; NM_001329120.2; NM_001127596.2; NM_001386450.1; NM_001098175.2; NM_001164178.1; NM_001320916.1; NM_001164181.1; NM_001164182.2; NM_001164183.2; NM_001312654.1; NM_001164179.2; NM 001776.6; NM_002838.5; NM_080921.4; NM_001267798.2; NM_001242608.2; NM_001400603.1; NM_001400606.1; NM_000615.7; NM_001386292.1; NM_001076682.4; NM_001386290.1; NM_001400615.1; NM_181351.5; NM_001242607.2; NM_001386289.1; NM_001386291.1; NM_001400618.1; NM_001400605.1; NM_001400616.1; NM_018644.3; NM_001367973.1; NM_054025.3; NM_001781.2; NM_001204813.2; NM_002526.4; NM_057157.2; NM_000783.4; NM_001303618.2; NM_006566.4; NM_001303619.2; NM_000639.3; NM_000639.3; NM_006433.5; NM_012483.4; NM_001302758.2; NM_006144.4; NM_004131.6; NM_001346011.2; NM_000211.5; NM_001303238.2; NM_001127491.3; NM_002417.5; NM_001145966.2; NM_014218.3; NM_013289.3; NM_002258.3; NM_001199805.1; NM_001199805.1; NM_001145466.2; NM_147130.3; NM_001145467.2; NM_001242356.3; NM_001242357.3; NM_001145458.3; NM_004829.7; NM_001145457.3; NM_003810.4; NM_001190942.2; NM_001190943.2; NM_001083116.3; and NM_005041.6.

Optionally, the activated cell does not have an expression of at least one gene, or gene expression product expressed thereby, selected from CD3, CD3G, CD3D, and CD3E.

Optionally, the activated cell does not have an expression of at least one gene having an Ensembl transcript ID, or gene expression product expressed thereby, selected from ENSG00000160654; ENSG00000167286; and ENSG00000198851.

Optionally, the activated cell does not have an expression of at least one gene having a Genbank accession/version number, or gene expression product expressed thereby, selected from NM_000073.3; NM_000732.6; NM_001040651.2; and NM_000733.4.

Optionally, the activated cell has an expression of at least one cytokine selected from IFN-gamma, GM-CSF, TNF, IL-10, IL-5, IL-13, CCL5, IL-8, and MIP1a&b.

Optionally, the activated cell has a higher expression of at least one cytokine relative to a non-activated cell (a cell not contacted with the at least one cytokine).

Optionally, the at least one cytokine is selected from IFN-gamma, GM-CSF, TNF, IL-10, IL-5, IL-13, CCL5, IL-8, and MIP1a&b.

Optionally, the step of providing a population of cells comprises isolating cells from splenic issue.

Optionally, the step of providing a population of cells comprises isolating cells from a population of splenocytes.

Optionally, the step of providing a population of cells comprises isolating cells from blood, optionally whole blood, optionally cord blood (blood obtainable from the placenta and/or umbilical cord).

Optionally, the step of providing a population of cells comprises isolating cells from a population of peripheral blood cells.

Optionally, the step of providing a population of cells comprises isolating cells from a population of peripheral blood mononuclear cells.

### Brief description of the drawings

Embodiments of the invention will be described with reference to the appended non-limiting examples and the accompanying drawings in which:
Figure 1A is a graphical representation of nvAMD illustrating (a) a crosssection of an eye with AMD and (b) a graphical representation of the fundus or interior surface of an eye with nvAMD, wherein neovascularization occurs from the choroidal retinal vasculature that lies underneath the retina;
Figure 1B is a graphical representation of DR illustrating (a) a crosssection of an eye with DR and (b) a graphical representation of the fundus or interior surface of an eye with DR, wherein neovascularization occurs from the inner retinal vasculature that enter the eye through the optic nerve entrance;

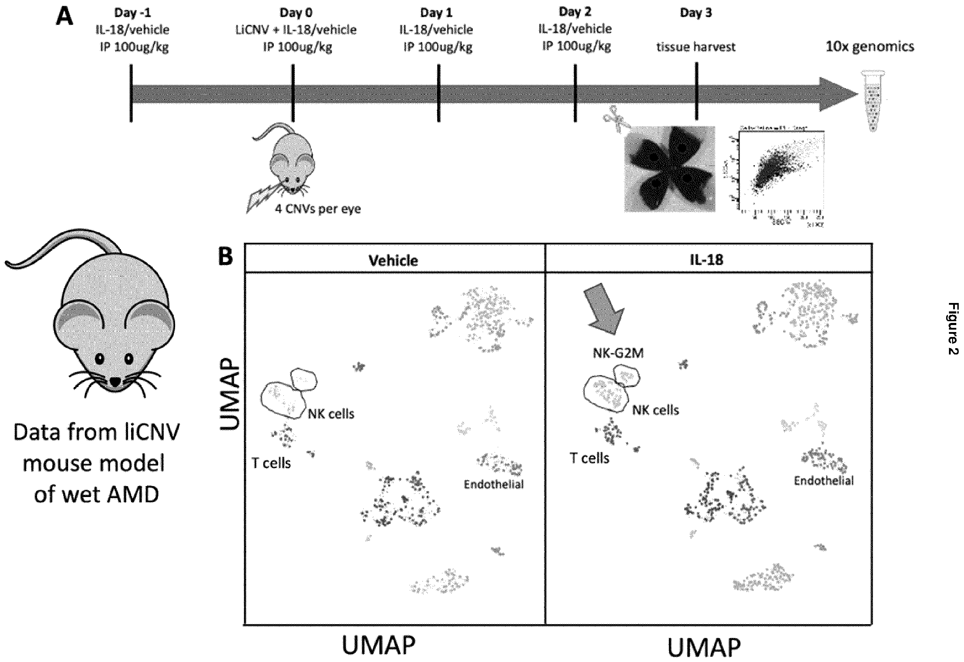

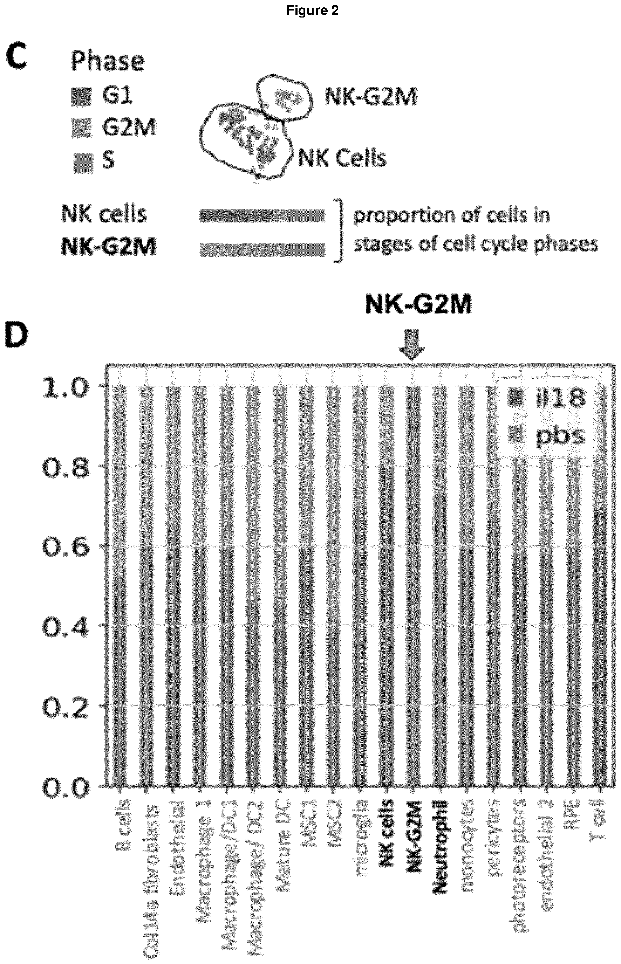

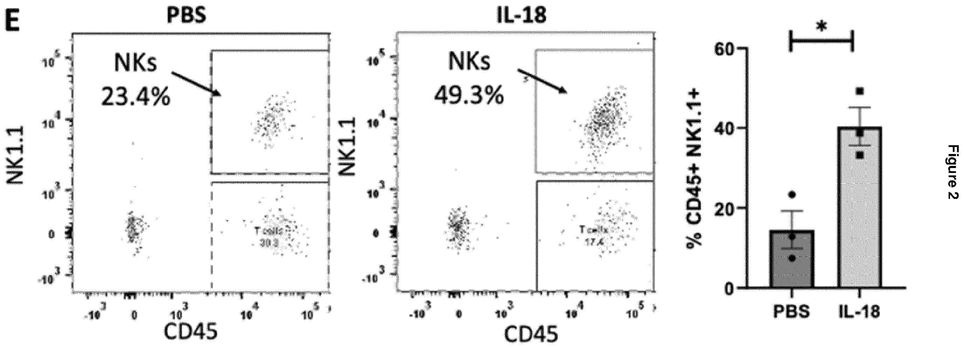

Figure 2 illustrates two defined clusters of NK cells found in mouse choroidal neovascular tissue, wherein A illustrates scRNA-sequencing experimental design for liCNV in mice; B illustrates UMAP of clusters (Scanpy analysis) of CNV tissue from vehicle and IL-18 treated mice (n=8); C illustrates cell cycle analysis of clusters identifies proliferating cluster of NK cells in IL-18 treated tissue termed NK-G2M; D illustrates presence of NK-G2M cells only found in IL-18 treatment, presence of proximal NK cells is proportionally higher in IL-18 treatment; and E illustrates scRNA seq NK cell data validated by Flow cytometry of CNV tissue from mice treated with vehicle or IL-18;

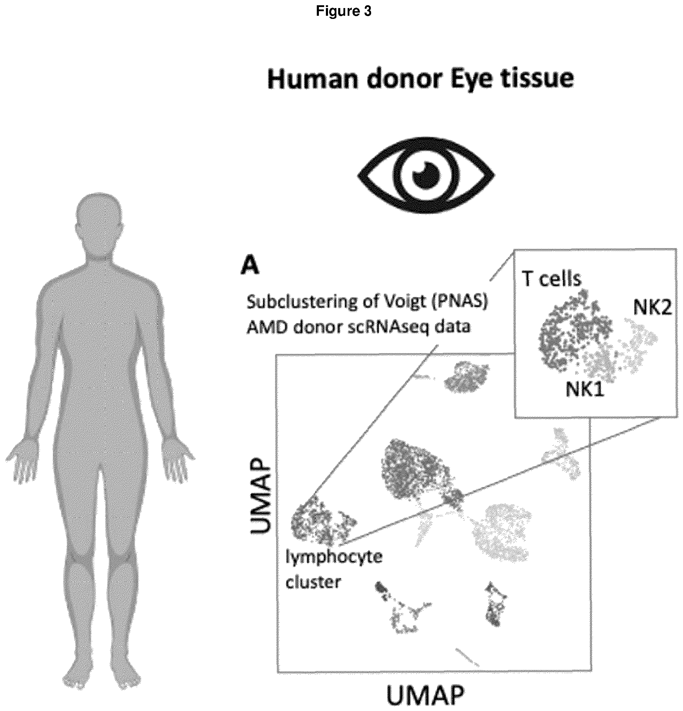

Figure 3 illustrates two defined clusters of NK cells found in human RPE/choroid tissue, whereby UMAP of cell clusters (Scanpy analysis) were re-analysed from scRNA-sequencing data of human RPE/choroid tissue show 2 NK cell clusters;

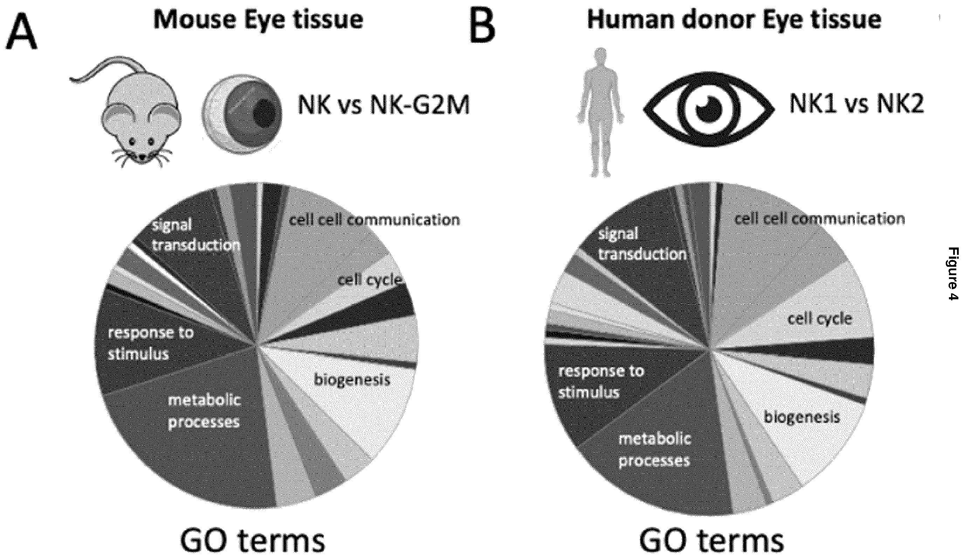

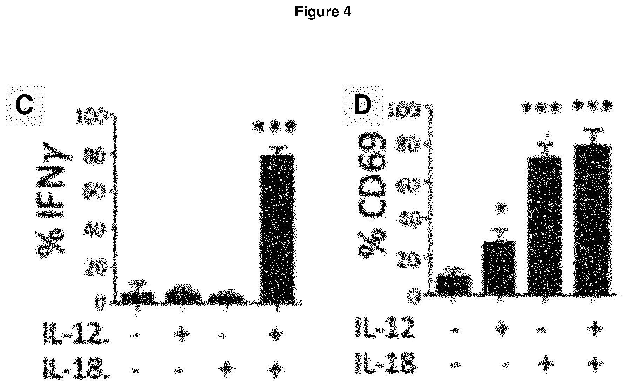

Figure 4 illustrates IL-18 directs metabolic processes to prime NK cells for expansion and migration, wherein panther GO-slim comparative analysis of biological processes indicated between NK cell subsets in mouse (A) and human (B) from 100 top DEGs; where in C illustrates IL-18 alone does not induce IFN-gamma but wherein D illustrates IL-18 does induce CD69 on NK cells isolated from splenocytes; and

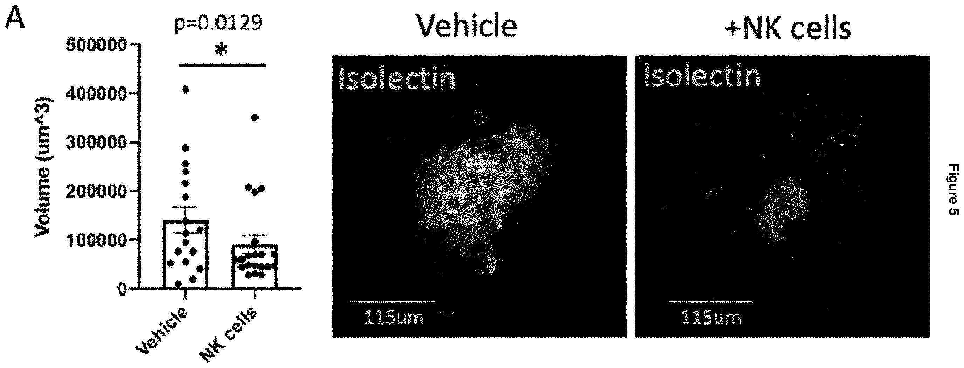

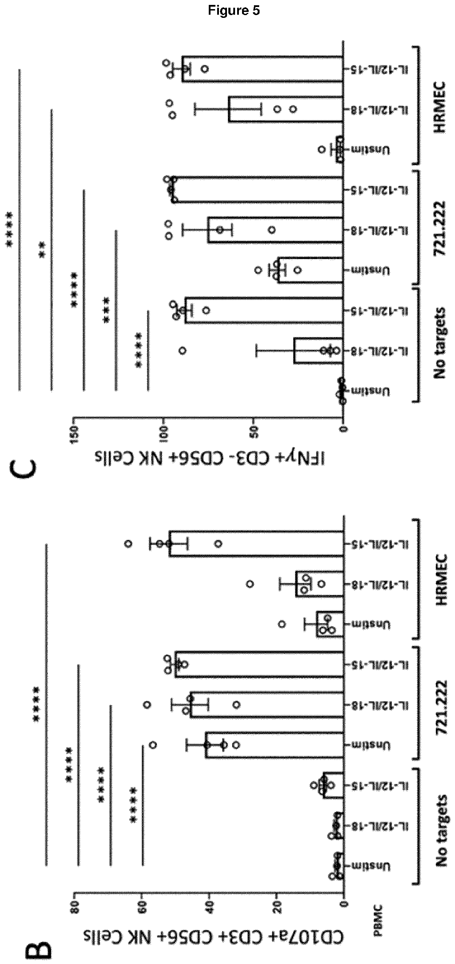

Figure 5 illustrates adoptive transfer of NK cells inhibits neovascularization in a liCNV model, wherein A illustrates at time of laser injury mice were injected ip with vehicle or NK cells (1x10e5) that had been activated with IL-18/IL-15/IL-12 for 18 h (n=6, per group) and 6 days later RPE flat mounts were stained for isolection (green) and CNV volume was analysed by confocal microscopy and IMARIS; B&C illustrate human retinal endothelial cells can act as target cells for NK cells, whereby PBMCs were cultured alone, with 721.211 target cells as positive control, or HRMECs as target cells and NK cells were assayed for expression of (B) CD107a and (C) IFN-gamma by FACS.

### Examples

### Materials and Methods

### Murine NK cells isolation and culture

NK cells were harvested from the spleens of C57BI/6J mice by magnetic labelling using the NK cell isolation kit as per manufacturer's instructions (Miltenyi Biotec). NK cells were cultured in RPMI 1640 medium (Sigma-Aldrich) supplemented with 10% FBS. Cells were activated with recombinant IL-18 (100 ng/ml), IL-12 (20 ng/ml) and IL-15 (20 ng/ml) overnight and then harvested and injected systemically into mice (5×10⁵ cells/mouse) post Li-CNV.

### Laser-induced choroidal neovascularization model

All mouse experiments were carried out in the Smurfit Institute of Genetics in TCD and adhere to the principles laid out by the internal ethics committee at TCD with national licenses obtained before commencement of studies. C57BI/6J mice were used for all experiments and animals were kept on a 12-h light/dark cycle. Laser-induced choroidal neovascularization (LiCNV) was induced in mice using a green 532-nm Iridex Iris laser (532 nm, 140 mW 100 mSec, 50 mm spot size, 3-6 laser spots per eye). For single cell RNA sequencing and flow cytometry experiments, mice were given daily intraperitoneal injections of recombinant mouse IL-18 (100 mg/kg) or PBS control for 4 consecutive days, starting a day prior LiCNV induction and mice were euthanized 3 days post LiCNV induction. For NK cell adoptive transfer experiments, mice were injected systemically with activated NK cells (5×10⁵ cells/mouse) or vehicle (RPMI 1640 media) immediately after LiCNV induction and mice were euthanized 7 days post LiCNV induction.

### Immunohistochemistry

Ocular tissue was harvested from freshly euthanised mice 7 days post LiCNV induction. Eyes were enucleated and the cornea and lens were carefully removed. Retinas were carefully separated from the RPE/choroid, and four incisions were made into the RPE/choroid to flatten the tissue. The RPE flat mounts were fixed in 4% PFA for 30 mins. After 3 PBS washes, the tissue was incubated with Griffonia simplicifolia isolectin Alexa-488 (Molecular Probes) (1:300) overnight at 4 °C. Following 3 PBS washes, the RPE flatmounts were mounted with Hydromount (VWR) mounting medium. CNV lesions were imaged using a confocal microscope (Zeiss LSM 710) and quantified using IMARIS software.

### Single cell RNA sequencing of excised liCNV tissue

### Tissue dissociation

Ocular tissue was harvested from freshly euthanised mice 3 days post LiCNV induction. Eyes were enucleated and the cornea and lens were carefully removed. Retinas were carefully separated from the RPE/choroid, and the RPE/choroid was digested in HBSS supplemented with 5% FBS, 10 mM HEPES, 1.5 mg/ml collagenase A and 0.1 mg/ml DNase I for 40 mins at 37°C with gentle agitation. The digested tissue was passed through a 70 mm filter to create a single cell suspension and washed in PBS containing 0.04% BSA. 7 eyes were pooled for each treatment group for single cell analysis, while 2 eyes were pooled for each n number for cell sorting.

### Fluorescence activated cell sorting

RPE/choroid single cell suspensions were stained with propidium iodide (5 mM for 5 mins) followed by Draq 5 (1 mg/ml for 5 mins) prior to FACS sorting. Live (PI^{-ve}), nucleated (Draq5^{+ve}) cells were sorted by FACS, and 20,000 cells were collected for each sample.

### scRNA-seq library preparation and sequencing

The sorted live cells were loaded onto the Chromium controller (10X Genomics) and the Chromium Next GEM Single Cell 3' Reagent Kits were used for library preparation as per manufacturer's instructions. Libraries were sequenced on Illumina HiSeq4000. Data was processed in Cell Rangeer and analysed using Seurat and Scanpy software.

### Flow cytometry analysis of Choroidal Neovascular mouse tissue

C57B/L6 mice with a laser induced CNV were injected I.P. with PBS or IL-18, as previously described. At the experimental endpoint, mice were sacrificed and retinal tissue homogenates were prepared. Cells of the retina were examined for surface expression of CD45 BB515 (clone 30-F11, BD) and NK1.1 BB700 (clone PK136, BD) as follows. Single cell suspensions were blocked with Mouse Fc block (clone 2.4G2, BD) and cells were stained with LIVE/DEAD^{™} Aqua (Molecular Probes), followed by staining for 20 m at 4°C, washed, and analysed by flow cytometry immediately. Flow cytometry was carried out on a BD LSR Fortessa cell analyzer and analyzed using FlowJo software (TreeStar).

### Re-clustering of human donor eye tissue single cell RNAseq data

scRNA sequencing data of human donor eye tissue from healthy and AMD patients deposited in Gene Expression Omnibus (GEO) database https://www.ncbi.nlm.nih.gov/geo (accession no. GSE135922) was processed using CellRanger software. Re-analysis of cell clustering and generation of UMAPs was carried out in Scanpy. Original dataset citation: (Voigt AP, Mulfaul K, Mullin NK, Flamme-Wiese MJ, Giacalone JC, Stone EM, Tucker BA, Scheetz TE, Mullins RF. Singlecell transcriptomics of the human retinal pigment epithelium and choroid in health and macular degeneration. Proc Natl Acad Sci USA. 2019 Nov 26;116(48):24100-24107. doi: 10.1073/pnas.1914143116. Epub 2019 Nov 11. PMID: 31712411; PMCID: PMC6883845.) Subclustering analysis revealed 3 distinct clusters in the lymphocyte cluster termed T cells, NK1 and NK2.

### Gene Ontology Analysis of NK cells

Analysis of differentially expressed genes between cluster NK1 and NK2 from the human GEO Dataset GSE13592 and between NK and NK-G2M mouse subclusters was undertaken using Gene Ontology (GO) enrichment analysis and the PANTHER
(**P**rotein **AN**alysis **TH**rough **E**volutionary **R**elationships) Classification System. GO aspect "biological process" was selected for analysis and expressed in pie chart. (Ashburner et al. Gene ontology: tool for the unification of biology. Nat Genet. May 2000;25(1):25-9.) (The Gene Ontology resource: enriching a Gold mine. Nucleic Acids Res. Jan 2021;49(D1):D325-D334.)

### Human CD107a assay

Protocol adapted from Brennan et al., 2016. Primary PBMCs were seeded at a density of 1 × 10⁶ cells per well in a round-bottomed 96 well plate and stimulated as required overnight. For 721.221 as targets the cells were freshly re-suspended and added to PBMCs (10:1 E:T ratio). For primary human retinal microvascular endothelial cells (HRMEC) (Innoprot) as targets the HRMEC were seeded at 4 × 10⁴ and incubated for 48 hours in 96 well plates prior to the addition the treated PBMCs. Anti-CD107a-APC (Biolegend) was added at a concentration of 2.5 µL per well. Plates were then centrifuged at 150 × g for 5 min and incubated for 1 hour before the addition of 10 µM of monensin (biolegend) for a further 3 hours. All incubations were performed at 37°C. Extracellular staining was performed with anti-CD3-FITC and anti-CD56-PECy7 (both Biolegend) and flow cytometric analysis was carried out with a BD FACS CANTO II and FlowJo software.

### Intracellular IFNγ staining

Cells were stimulated as required with the final 3 hours of the stimulation including the addition of Brefeldin (Invitrogen). Cells were washed in PBS and FcRs blocked by incubating with 10% human AB serum/1% FCS/PBS. Cell surface staining performed as above followed by intracellular staining with IFN-γ-PE (Biolegend) using the Foxp3 kit (Invitrogen) as per instructions provided.

### Mouse CD69 and IFNgamma Assays for NK cell activity

Spleens were dissected from C57BL/6J mice. NK cells in C57BU6J mouse splenocytes (gated on NKp46+NK1.1+CD3- NK cells) were stimulated with IL-18 (25 ng/ml, 18 hours, SB-528775) with or without IL-12 (10 ng/ml, R&D). CD69+ and intracellular IFN-gamma were analysed by flow cytometry. Flow cytometry was carried out on a BD LSR Fortessa cell analyser and analysed using FlowJo software (TreeStar). Data are means ± SEM (n = 5 mice, three independent experiments). P values were determined by analysis of variance (ANOVA) with Dunnett's multiple comparison test.

### Example 1

### IL-18 metabolically rewires NK cells for population expansion

Cytokine signalling is crucial for activating NK cell responses via signal transducer and activator of transcription (STAT) proteins, and cytokine pairs IL-12/IL-18, IL-2/IL-12 or IL-2/IL-15 can be NK cell activators with each pair initiating a unique signalling pathway resulting in alternative repertoires of STAT activation. The present data indicate that, *in vivo* in mice, systemically administered rIL-18 alone primed NK cells for proliferation, with the most differentially expressed genes (DEGs) in the NK cell clusters being *top2a*,which peaks in G2M; *pclaf* a cell cycle protein known to correlate with proliferation and *Smc2* a marker of mitosis, i.e. genes involved in cell-cycle regulation and proliferation. In fact, when gene ontology (GO) term enrichment analysis was carried out on the 100 most highly DEGs between NK and NK-G2M, to determine how they might differ in respect of function, we found that in addition to cell-cycle regulatory genes, NK-G2M were enriched with genes involved in metabolic rewiring processes and biogenesis (Fig 4A, depicted in pie chart format). In an intriguing parallel, when the same GO analysis was carried out on the 100 most highly DEGs between the two NK clusters from the human donor tissue, almost identical hierarchy of enriched biological processes was observed (Fig 4B) indicating that these cells are most likely relevant for human disease also, and that the separate clusters most likely represent quiescent vs proliferating NK cell populations.

In parallel with the *in vivo* data indicating rIL-18 metabolically rewires NK cells for population expansion, *in vitro,* NK cells derived from mouse splenocytes treated with rIL-18 alone are also metabolically rewired to take on an energetic phenotype to a similar degree as the IL-2/IL-12 pairing as assayed by Agilent Seahorse XF Cell energy phenotype stress test (Fig. 4C). Of note, while unable to induce IFNy (Fig. 4D), rIL-18 alone does induce CD69 (Fig. 4E). CD69 is an early marker of lymphocyte activation and, importantly, a costimulatory molecule that promotes migration and proliferation of NK cells and increases mTOR signalling. Altogether, these data provide strong evidence for a role for IL-18 in directing metabolic processes to prime NK cells for expansion and subsequent migration.

### Example 2

### Adoptive transfer of activated NK cells reduces choroidal neovascularization in vivo

NK cells isolated from splenocytes were activated with the cytokine cocktail IL-18/IL-15/IL-12 and injected intraperitoneally on the same day laser-induced injury was performed (Fig. 5A). It is clear that transfer of autologous, activated NK cells reduced laser-induced choroidal neovascularization (liCNV) 6 days post injury. In this model a laser creates a perforation in Bruch membrane allowing the choroidal vasculature to grow into the sub-retinal space. The advantage of this model is it is inducible and acute with final tissue collection complete 6 days post laser injury. It is also amenable to the method of cytokine expression or use of recombinant cytokines. Furthermore, it appears that activated NK cells can interact directly with endothelial cells evidenced by expression of CD107a on NK cells when cultured with human retinal endothelial cells (HRMECs) as target cells (Fig 5B,C last 3 bars).

Without being bound, by theory, it is believed that the NK-G2M cell infiltrate observed with rIL-18 treatment is involved in a reparative vascular remodelling process. Despite being best known for their cell-directed cytotoxicity, NK cells play a critical regulatory role at the foetal-maternal interface during the first trimester of pregnancy, constituting 50-90% of total lymphocytes in the uterus, and are responsible for remodelling the vasculature and establishing an immune modulatory environment for survival of the foetus. Subsets of NK cells also play a regulatory role in the liver, where they maintain homeostasis and enable tissue regeneration after injury. Additionally, NK cells are described to play a role in limiting tissue damage through immune editing, i.e. cytotoxic killing of immune cells including immature DC, macrophages, activated T cells and eosinophils. There are a range of diseases termed Hemophagocytic lymphohistiocytosis (HLH) syndromes caused by mutations in the cytolytic machinery used by NK cells. A consistent finding in HLH is that NK cell cytotoxic capabilities are low to absent. While these individuals are susceptible to viral infection, they also have widespread inflammation and are treated with immunosuppressants. Of note, it is mutations in the perforin gene, a pore-forming protein that polymerises on the plasma membrane of target cells to introduce cytotoxic effector granules that account for approximately 40% of HLH patients. The cause of the widespread inflammation is due to loss of NK cell mediated cytolysis of inflammatory cells including macrophages and T cells, and subsequent failure to regulate or terminate an immune response.

Thus, NK cells act in a regulatory manner, playing a role in limiting tissue damage, in various specific contexts. It is also worth noting, in the context of the retina specifically, that neutrophils, another cytotoxic cell type, have been shown to be involved in the remodelling of the retinal vasculature during oxygen induced retinopathy. This preliminary dataset makes it clear that NK cells must inevitably impact retinal neovascularization, that this transcends species.

The current state-of-the-art for almost all studies related to examining sterile retinal neovascular disease is to focus on innate immune myeloid cells such as microglia, monocytes, macrophages, mast cells, neutrophils, or others. The present invention takes an alternative approach and examine the role of an innate immune lymphoid cell population at the neovascular-retinal interface. It has been shown (a) that in addition to innate myeloid cells, innate lymphoid NK cells are found in neovascular tissue, (b) that their *in vivo* expansion coincides with the administration of a therapeutic cytokine known to ameliorate neovascular disease and vascular permeability in the retina and (c) that when NK cells are transferred at the same time as liCNV in a model of wet AMD, the neovascular lesions are smaller. Specifically, NK cell activating cytokine cocktails of IL-12, IL-18, IL-2 and or IL-15 inhibit neovascularization and vascular permeability, supporting the hypothesis that combinations of these cytokines will potently inhibit pathological retinal neovascularization and vascular permeability.

Innate lymphoid cells (ILCs), however, are an understudied immune cell population in retinal degenerative disease in general. Natural Killer cells (NK cells) are an ILC population of cells, most closely related to group 1 ILCs due to their IL-18 responsiveness and ability to produce interferongamma (IFN-gamma). However, unlike group 1 ILC's, NK cells have direct cell-mediated cytolytic activity against physiologically stressed cells, and as such they play important roles in immune defence against virus infected cells and tumour. Remarkably, despite a long reported association between HLA class 1 Cw*0701 allele and NK cell immunoglobulin-like receptor (KIR) haplotype AA with AMD, and CNV in particular; NK cells' role in retinal neovascular disease is completely unknown.

The effects of IL-18 on neovascularization appear to be context-dependent because, while it also inhibits neovascularization in tissues other than the eye, it is proangiogenic in joint synovium. However, despite initial controversy disputing the ability of IL-18 to regulate retinal neovascularization, data have confirmed that IL-18 is anti-angiogenic and protects against vascular permeability in the retina in vivo. The anti-angiogenic, anti-permeability function of IL-18 in vivo in has also been demonstrated in models of ocular neovascularization. IL-18 has been shown to be an anti-angiogenic cytokine which suppresses corneal neovascularization, and IL-18 knockout mice have been shown to have abnormal retinal vascular development, and that compared to wild-type mice IL-18 deficiency worsens oxygen-induced retinopathy (OIR) this is a hypoxia driven angiogenesis model most widely used to model ischemic retinopathies. One advantage of this model is the established precise timing of vaso-obliteration and neovascularization and vascular regression. Exposure to hyperoxic conditions (75 % O2) in mice from P7 to P12 induces an initial phase of vascular dropout (vaso-obliteration). Upon return to room air at P12, ischemia triggers pathological preretinal neovascularization that peaks at P17. From p17 to p21, preretinal neovascularization regresses. This is consistent with data demonstrating worsened CNV in IL-18 knockout mice compared to wildtype mice. It has also been demonstrated that administration of rIL-18 reduced vascular leakage and ischemia-induced retinal neovascularization. The reduced vascular leakage observed in models of choroidal and retinal neovascular disease is also consistent with an observation that IL-18 has an anti-permeability effect in rat brain where it reduces oedema associated with status epilepticus.

IL-18 can modulate the function of a variety of cell types that are found in neovascular lesions and, in the present examples, to pinpoint specific cell types involved in the promotion of vascular integrity, single cell RNA (scRNA) sequencing of the tissue surrounding neovascular lesions was undertaken in mice administered intraperitoneal rIL-18 therapy or vehicle. Three days post iiCNV, the tissue around the neovascular lesions was excised and prepared for 10X genomics scRNA sequencing. After QC filtering, 19 clusters were identified. Strikingly one cluster was found only in the tissue isolated from the rIL-18 treated mice. Closer analysis of this cluster of cells demonstrated that these were a defined cluster of NK cells that differed from the proximal "NK cell" cluster mainly due to their cell cycle phase, ribosome biogenesis and metabolic activity. As such they are a defined, proliferating NK cell cluster, referred to herein as "NK-G2M". Of note, in addition to the appearance of these NK-G2M cells, it was evident that the proportion of NK cells in the proximal cluster was also increased with rIL-18 treatment compared with vehicle control. This increased presence of NK cells in neovascular tissue observed in response to rIL-18 in our scRNA sequencing experiment was confirmed by flow cytometry, where NK cells made up ~50 % of the CD45+ leukocyte population in the CNV tissue isolated from mice treated with rIL-18, double the NK cells present in the vehicle control.

To investigate whether these findings in the liCNV mouse model were translatable and could apply to the human condition, a scRNA-seq dataset from human AMD donor eye tissue samples in which a large lymphocyte cluster was identified was analysed. On closer analysis, 3 sub-clusters were identified within this human lymphocyte cluster, mirroring the present mouse dataset (2 × NK cell clusters and 1 × T cell cluster). Furthermore, this tissue was obtained from non-disease and AMD donor eyes, and when analysed by disease state the NK cell groups clustered strongly with neovascular AMD.

## Claims

1. A method of producing a population of activated innate lymphoid cells, the method comprising the steps of:
(a) providing a population of innate lymphoid cells; and
(b) contacting the population of innate lymphoid cells with at least one cytokine.

2. A population of activated innate lymphoid cells obtainable by the method of claim 1.

3. A population of activated innate lymphoid cells of claim 2 for use in preventing or treating an ocular disease in a subject.

4. A method of claim 1, a population of activated innate lymphoid cells of claim 2, or a population of activated innate lymphoid cells for use of claim 3, wherein the at least one cytokine is selected from IL-18, interleukin 12, interleukin 15, interleukin 2, and combinations each thereof.

5. A method of claim 1 or 4, a population of activated innate lymphoid cells of claim 2 or 4, or a population of activated innate lymphoid cells for use of claim 3 or 4, wherein the at least one cytokine is IL-18.

6. A method of claim 1, 4 or 5, a population of activated innate lymphoid cells of claim 2, 4 or 5, or a population of activated innate lymphoid cells for use of any one of claims 3-5, further comprising the additional step of contacting the population of innate lymphoid cells with at least one cytokine selected from IL-15 and IL-2 to expand the population of innate lymphoid cells.

7. A population of activated innate lymphoid cells for use of any one of claims 3-6, wherein the ocular disease is a neovascular disease of the choroid and/or the retina.

8. A population of activated innate lymphoid cells for use of any one of claims 3-7, wherein the ocular disease is selected from age-related macular degeneration, retinopathy of prematurity, diabetic retinopathy, diabetic macular edema, and branch retinal vein occlusion.

9. A population of activated innate lymphoid cells for use of any one of claims 3-8, wherein the ocular disease is age-related macular degeneration.

10. A population of activated innate lymphoid cells for use of any one of claims 3-9, wherein the ocular disease is wet (neovascular) age-related macular degeneration.

11. A population of activated innate lymphoid cells for use of any one of claims 3-10, wherein the ocular disease is optionally choroidal neovascularization (CNV).

12. A method of claim 1 or 4-6, a population of activated innate lymphoid cells of claim 2 or 4-6, or a population of activated innate lymphoid cells for use of any one of claims 3-11, wherein the innate lymphoid cell is a natural killer cell.

13. A method of claim 1, 4-6 or 12, a population of activated innate lymphoid cells of claim 2, 4-6 or 12, or a population of activated innate lymphoid cells for use of any one of claims 3-12, wherein the activated cell is in a phase of the cell cycle selected from Gap 2 (G2) and the mitotic phase (mitosis, M).

14. A method of claim 1, 4-6, 12 or 13, a population of activated innate lymphoid cells of claim 2, 4-6, 12 or 13, or a population of activated innate lymphoid cells for use of any one of claims 3-13, wherein the step of providing a population of cells comprises isolating cells from splenic issue, a population of splenocytes, blood, or a population of peripheral blood mononuclear cells.
